# EUROPEAN PATENT SPECIFICATION

(11) **EP 1 393 701 B1**
(45) Date of publication and mention of the grant of the patent: **24.07.2013**
(21) Application number: 03102637.0
(22) Date of filing: 22.08.2003
(51) Int. Cl.: A61F 13/15

(54) **Method and apparatus for producing wearing article**
Verfahren und Vorrichtung zur Herstellung eines tragbaren Artikels
Méthod et appareil pour produire un vêtement

(30) Priority: 22.08.2002 JP 2002241959; 28.10.2002 JP 2002312403; 26.11.2002 JP 2002342073
(43) Date of publication of application: 03.03.2004
(73) Proprietor: Zuiko Corporation, Osaka 566-0045 (JP)
(72) Inventor: Nakakado, Masaki, Osaka, Osaka 573-0065 (JP); Satoh, Hitoshi, Osaka, Osaka 560-0034 (JP); Tanaka, Satoshi, Osaka, Osaka 563-0043 (JP); Tachibana, Ikuo, Osaka, Osaka 662-0072 (JP)
(74) Representative: Suckling, Andrew Michael

(56) References cited:
- EP-A- 0 211 197
- EP-A- 0 974 323
- EP-A- 1 004 285
- US-A- 4 642 150
- US-A- 4 770 657
- US-A- 4 886 512
- US-A- 5 312 386
- US-A- 5 743 994
- PATENT ABSTRACTS OF JAPAN vol. 0172, no. 70 (C-1063), 26 May 1993 (1993-05-26) & JP 5 007605 A (KAO CORP), 19 January 1993 (1993-01-19)

## Description

### BACKGROUND OF THE INVENTION

### 1. Field of the Invention

The present invention relates to a method and apparatus for producing a disposable wearing article such as paper diapers and pants.

### 2. Description of the Related Art

In the above-mentioned type of wearing articles, in order to form, for example, a waist gathering, an elastic member is placed on a sheet surface. However, such an elastic member allows an absorber to shrink, so that the absorber becomes stiff, degrading a feeling of wearing.

As a prior art of cutting an elastic member, Japanese Laid-Open Patent Publication No. 2001―224627 discloses a method for interposing a film sheet for gathering between a peripheral surface of an adsorbent roller and a cutter blade of a cutter roller, and cutting the film sheet.

Furthermore, International publication No. WO 00/04855 discloses a method for forming a web loop.

US 5 743 994 discloses a method and apparatus for making an elasticized article. Discrete elastic members are attached to a substrate web, by a first connecting mechanism attaching an inboard elastic member to the substrate web and a second connecting mechanism attaching an outboard elastic member to the substrate web, with the outboard elastic member located laterally outwards of the inboard elastic member. A dividing member separates away a portion of the substrate web and a portion of the outboard elastic member to thereby provide a set of elastics which are arranged in staggered overlapping relationship.

### Summary of the Invention

In order to achieve the above-mentioned object, a method for producing a wearing article of the present invention includes the steps of: supplying an elastic member; stretching the elastic member; placing the stretched elastic member so that the elastic member spreads across a plurality of first webs divided in a transport direction; making a part of a second web loose in the transport direction while transporting the second web, thereby forming a loose portion; placing the first webs, on which the elastic member is disposed, on non-loose portions before and after the loose portion of the second web and placing an absorber on a region of the second web between adjacent first webs with the elastic member cut therebetween; cutting the elastic member between adjacent first webs of the plurality of first webs such that the elastic member is between the first webs and the second web, but not placing the first webs, on which the elastic member is disposed, on the loose portion of the second web.

According to the present invention, the loose portion formed in the second web may be folded into two to form a folded portion. Furthermore, after the first webs are placed, and before and/or after the elastic member is cut, the loose portion of the second web is stretched so as to position the first webs away from each other.

A second aspect of the invention provides an apparatus for producing a wearing article, the apparatus comprising: means for supplying an elastic member; means for stretching the elastic member; means for placing the stretched elastic member so that the elastic member spreads across a plurality of first webs divided in a transport direction; means for making a part of a second web loose in the transport direction while transporting the second web, thereby forming a loose portion; means for placing the first webs, on which the elastic member is disposed, on non-loose portions before and after the loose portion of the second web such that the elastic member is between the first webs and the second web, but not placing the first webs, on which the elastic member is disposed, on the loose portion of the second web; and a cutter for cutting the elastic member between adjacent first webs of the plurality of first webs; and means for placing an absorber on a region of the second web between adjacent first webs with the elastic member cut therebetween.

In a preferred embodiment the apparatus further comprises: a roller for supplying the plurality of first webs divided in a transport direction at a predetermined position in a state where the elastic member is placed so as to spread across the first webs; and a rotation apparatus including a plurality of pads which are rotated to continuously transport the second web and approach the roller at the predetermined position, wherein the respective pads of the rotation apparatus have varying circumferential velocities,
wherein the respective pads of the rotation apparatus receive the second web at a receiving position upstream from the predetermined position, and after receiving, an interval between the adjacent pads is decreased while the pads are rotated to the predetermined position, whereby the loose portion is formed in the second web,
wherein the first webs are placed to be stacked on a surface of the second web on a basis of the pads at the predetermined position,
wherein an interval between the adjacent pads is increased while the respective pads of the rotation apparatus are rotated from the predetermined position to a releasing position downstream from the predetermined position, and
wherein the cutter cuts, in use, the elastic member in the loose portion while the pads are rotated from the predetermined position to the releasing position.

As the rotation apparatus, for example, a rotation apparatus described in U.S. Patent 6722494 may be used.

A rotation apparatus disclosed in U.S. Patent Application Publication No. US2002/0103468 may also be used. The disclosures of these are incorporated herein by reference.

According to the present invention, an absorber may be placed between a plurality of first webs with the elastic member cut therebetween. In this case, a shrinking force does not act on an absorber, so that the absorber does not become stiff.

As the elastic member in the present invention, a material containing thread rubber, flat rubber, mesh-shaped rubber, a film, or a thermoplastic elastic member may be adopted. As a material containing a thermoplastic elastic member, hot-melt resin is considered. Furthermore, a plurality of holes or slits may be formed in a film.

According to the present invention, the elastic member may be cut with a light cutter. The light cutter may cut the elastic member by irradiating the elastic member between the plurality of first webs with infrared rays and UV-rays. Furthermore, the elastic member may be cut with an ultrasonic wave.

The wearing article may include products or semi-finished products of a wearing article such as disposable diapers, disposable pants, bandages or the like. The wearing article may further include a single body and a stack of sheets such as fabric, non-woven fabric, a liquid-transparent sheet, or a non-liquid-transparent sheet.

The heat capacity of the stretched edge of the web is small. Therefore, a transport velocity of the web is increased by heating the edge, whereby a transport amount per unit time can be increased. In other words, a heating time for heating the edge can be prolonged in accordance with a ratio at which the edge of the web is stretched. Therefore, even when the transport velocity of the web is large, heat-sealing can be conducted exactly.

These and other advantages of the present invention will become apparent to those skilled in the art upon reading and understanding the following detailed description with reference to the accompanying figures.

### BRIEF DESCRIPTION OF THE DRAWINGS

FIG. **1** is a side layout view of an apparatus for producing a wearing article of Embodiment 1 according to the present invention.
FIG. **2** is a side layout view illustrating the steps of cutting an elastic member in the apparatus shown in FIG. **1**.
FIG. **3A** is a perspective view of a stack in a state before the elastic member is cut, and FIG. **3B** is a perspective view of a stack in a state after the elastic member is cut.
FIG. **4A** is a front view showing the stack in which the elastic member interposed between webs is cut, and FIG. **4B** is a front view showing a wearing article.
FIGS. **5A** and **5B** are partial side views showing a circumferential velocity of pads.
FIG. **6** is a side layout view showing an apparatus for producing a wearing article of Embodiment 2 according to the present invention.
FIG. **7** is a front view showing webs in a state where the elastic member is placed.
FIG. **8** is a front view showing webs in a state where an absorber is attached.
FIG. **9A** shows webs whose both edges are bent, and FIG. **9B** shows webs folded into two.
FIGS. **10A** to **10F** are front views illustrating a method for placing a second elastic member.
FIGS. **11A** to **11C** show an example of cutting the elastic member with a cutting machine, and FIG. **11D** shows another cutting machine. FIG. **11E** shows another rotatable small roller.
FIG. **12** shows diapers obtained by cutting the elastic member so that a trim is not generated, and shifting the phase of one web thus cut by substantially ½ from the other web.

### DESCRIPTION OF THE PREFERRED EMBODIMENTS

Hereinafter, the present invention will be described by way of illustrative embodiments with reference to the drawings.

### Embodiment 1

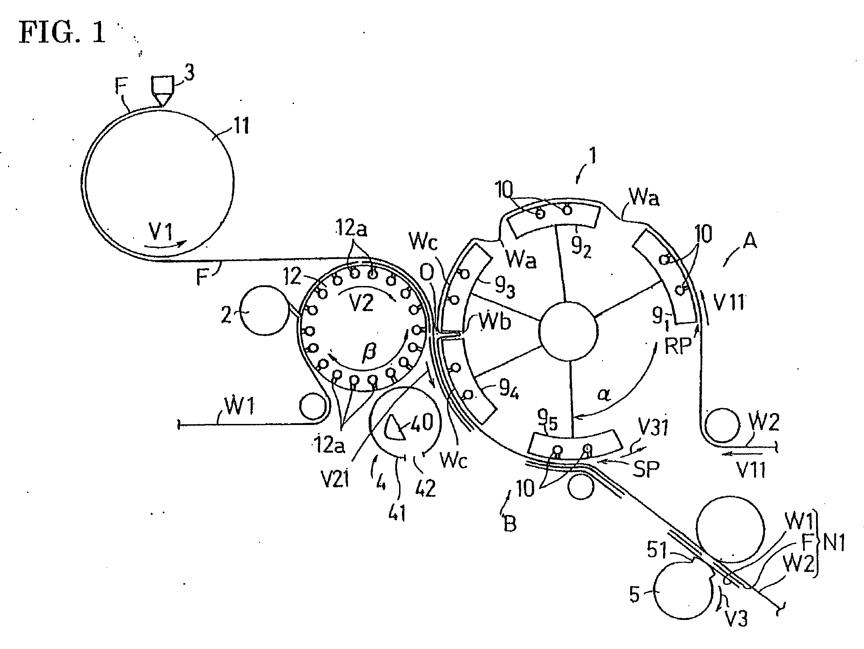

FIGS. **1** to **5A-B** show Embodiment 1.

An apparatus for producing a wearing article shown in FIG. **1** is capable of providing a web with looseness and intermittently placing an elastic member on at least a part of the web. For example, the apparatus for producing a wearing article includes a rotation apparatus **1** capable of providing a second web **W2** with looseness and a second roller **12** capable of placing an elastic member **F** on portions of the second web **W2** other than those which are loose. When the elastic member **F** extending across a loose portion of the second web **W2** is cut with at least one of a laser, an industrial light, a blade, and scissors, the apparatus can intermittently place the elastic member **F** on the second web **W2**. This intermittent placement is mainly caused by the looseness of the second web **W2.** Hereinafter, the configuration and operation of the rotation apparatus **1**, the second roller **12**, and the like will be described.

In FIG. **1**, a supply apparatus **3** of the elastic member **F** supplies the elastic member **F** to the surface of a first roller **11**. The elastic member **F** on the first roller **11** is fed to the second roller **12**. A circumferential velocity **V2** of the second roller **12** is set to be larger than a circumferential velocity **V1** of the first roller **11**. Therefore, the elastic member **F** is stretched between the first roller **11** and the second roller **12**.

The second roller **12** is supplied with a first web **W1**. The second roller **12** transports the first web **W1** while sucking the first web **W1** by vacuum suction or the like. On the surface of the second roller **12**, for example, a number of suction holes **12a** for sucking the first web **W1** may be provided. After the first web **W1** is sucked to be held on the surface of the second roller **12** by vacuum suction or the like, the first web **W1** is divided into a predetermined size with a web cutter **2**. In this division, the first web **W1** may be cut or half-cut to a predetermined length in a transport direction. In the case where the first web **W1** is half-cut, the first web **W1** may be cut to a predetermined length together with the elastic member **F** in a subsequent step or cut in a predetermined length when an interval of pads is increased. After this division, the elastic member **F** is placed so as to spread across the surface of the respective first webs **W1**. In the case where the elastic member **F** is made of thread rubber or flat rubber, an adhesive is applied to at least one of the elastic member **F**, the first web **W1**, and the second web.

It may also be possible that after the elastic member **F** is placed on the first web **W1**, the elastic member **F** is cut together with the first web **W1.** Furthermore, in the case where the elastic member **F** is made of hot-melt resin, molten resin discharged from the supply apparatus **3** is cooled by the first roller **11**. The molten resin may be cooled with cool water. Furthermore, the first roller **11** may be a porous roller. In the case where the first roller **11** is a porous roller, the first roller **11** may be configured so that air is discharged from the inside of the first roller **11** through holes. Due to the air, the hot-melt resin becomes unlikely to adhere to the roller. When the air is discharged from the porous body, the volume of the air is increased to cool the roller. Thus, the hot-melt resin can be cooled. The porous roller may be formed by sintering stainless ball or the like.

Furthermore, it may also be possible that the first roller **11** is provided with grooves or the like, whereby resin is molded. For example, mesh-shaped (for example, lattice-shaped) grooves are formed on the first roller **11**, and hot-melt resin is applied to the grooves of the first roller **11** with a coater or the like, whereby a mesh-shaped elastic member is formed.

When hot-melt resin having a softening point lower than that of Lycra ® is used as the elastic member, the subsequent cutting step becomes easier, compared with the case of using Lycra ®.

The hot-melt resin may be a kneaded composition and may comprise a thermoplastic resin, tackifier, viscosity adjuster, antioxidant, heat stabilizer, ultraviolet radiation absorbent, bulking agent, elasticizer, and the like. The thermoplastic resin may include elastomers such as olefins {EVA (ethylene-vinyl acetate copolymer), APAO (amorphous-poly-a-olefin)}, rubbers {SIS (styrene-isoprene -styrene copolymer), SBS (styrene-butadiene-styrene copolymer), SEBS (styrene-ethylene-butadiene-styrene copolymer), SEPS (styrene-ethylene-propylene-styrene copolymer)}, polyamides and polyesters. The thermoplastic resin may be a single elastomer or a blend of more than one elastomer.

The rotation apparatus **1** for folding is placed in the vicinity of the second roller **12.** The rotation apparatus **1** has a plurality of pads **9** capable of sucking the second web **W2** and transporting it. On the surface of a pad **9ᵢ**, suction holes **10** for sucking the second web **W2** are provided. The rotation apparatus **1** continuously transports the second web **W2**, and is in contact with the surface of the second roller **12** via the first web **W1**, the elastic member **F**, and the second web **W2** at a contact point **O** (predetermined position) . Thereafter, the following may also be possible: an additional roller **50** and the pad **9ᵢ** sandwich the first web **W1**, the elastic member F, and the second web **W2** placed on the pad **9ᵢ**, whereby the contact therebetween is enhanced.

The elastic member **F** is cut after being interposed between the first web **W1** and the second web **W2.** The elastic member **F** is fixed between the webs **W1** and **W2**, so that the elastic member **F** shrinks less, compared with the case where the elastic member **F** is placed on one web.

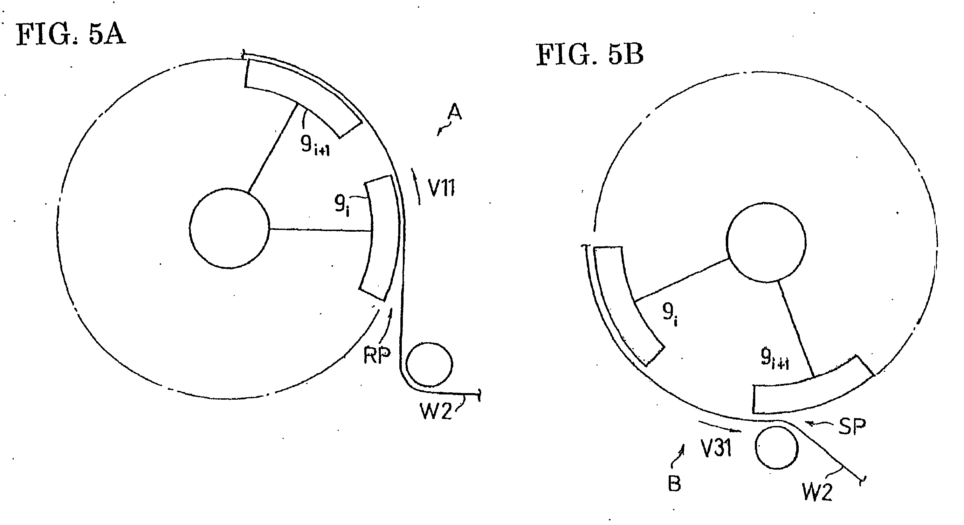

The rotation apparatus **1** receives the second web **W2** at a circumferential velocity **V11** at a receiving position **RP**. At a point "**A**" where the pad **9ᵢ** receives the second web **W2**, the circumferential velocity of the pad is **V11**. FIG. **5A** is a side view showing the enlarged vicinity of the point "**A**" of FIG. **1**. During a period from a time when at least a part of the pad **9ᵢ₊₁** shown in FIG. **5A** receives the second web **W2** to a time when at least a part of the pad **9ᵢ** adjacent to the pad **9ᵢ₊₁** receives the second web **W2**, the pad **9ᵢ₊₁** moves at a substantially constant circumferential velocity **V11**. The reason for this is to prevent the second web **W2** from being damaged.

Each pad **9ᵢ** of the rotation apparatus **1** is rotated at the receiving position **RP** at the circumferential velocity **V11**, and decelerates to a circumferential velocity **V21** before reaching the contact point **O** (where the distance between the pad **9ᵢ** and the pad **9ᵢ₊₁** becomes minimum). Therefore, the interval between the pad **9ᵢ** and the pad **9ᵢ₊₁** becomes narrow before the pad **9ᵢ** moves from the receiving position **RP** to the contact point **O**. This causes the second web **W2** to become loose between the pad **9ᵢ** and the pad **9ᵢ₊₁**, whereby a loose portion **Wa** is formed.

The pad **9ᵢ** comes into contact with the second roller **12** via the second web **W2** and the like at the circumferential velocity **V21** (**V11** > **V21**) at the contact point **O** shown in FIG. **1**. The first web **W1** is transferred from the second roller **12** at the circumferential velocity **V2**. The circumferential velocity **V21** of the pad **9ᵢ** is set at a substantially constant velocity equal to or close to the circumferential velocity **V2** of the second roller **12**. That is, while at least the pad **9ᵢ** is in contact with the second roller **12** via the second web and the like or at least the pad **9ᵢ** places the first web **W1** on the second web **W2,** the pad **9ᵢ** moves at the substantially constant circumferential velocity **V21** substantially equal to **V2**. In other words, during a period from a time when the second web **W2** on the pad **9ᵢ** starts receiving the first web **W1** to a time when the second web W2 finishes receiving it, the pad **9ᵢ** moves the constant circumferential velocity **V21** (≒**V2**). The difference in velocity prevents the first web **W1** and the second web **W2** from being shifted from each other, and exactly places the first web **W1** at a predetermined position of the second web **W2.**

The circumferential velocity of the pad **9ᵢ₊₁** becomes **V31** at a point **B** where the pad **9ᵢ₊₁** releases the second web **W2**. FIG. **5B** is a side view showing the enlarged vicinity of the point **B**. The pad **9ᵢ** moves at a substantially constant circumferential velocity **V31** at least during a period from a time slightly before the pad **9ᵢ₊₁** adjacent to the pad **9ᵢ** releases the second web **W2** to a time when the pad **9ᵢ** releases the second web **W2** (FIG. **5B**). The reason for this is to minimize the fluctuation in velocity of the released second web **W2**. For example, in the case where the circumferential velocity of an embossing roller **5** is **V3**, the circumferential velocity **V31** becomes substantially equal to **V3**.

When or after the circumferential velocity of the pad **9ᵢ** becomes maximum in the vicinity of the point **A** in FIG. **5A**, the pad **9ᵢ** receives the second web **W2.** If the pad **9ᵢ** receives the second web **W2** before the circumferential velocity of the pad **9ᵢ** becomes maximum, the interval between adjacent pads is increased, which may damage the second web **W2**. As the operation of the pad **9ᵢ**, the pad **9ᵢ** may move at a maximum circumferential velocity, for example, during a period from the point **B** to the point **A**. In this case, a region from the point **B** to the point A can be dealt with as one region. That is, in the present embodiment, the circumferential velocity of the pad may become substantially constant at least at two points.

It is preferable that the apparatus is provided with a directing part (not shown) so that the loose portion **Wa** in FIG. **1** becomes loose exactly toward the centre of the rotation apparatus. As the directing part, for example, a directing part as described in EP-A-1 234 787 may be used.

The directing part may be, for example, a mechanism for jetting air or a mechanism for pushing the loose portion **Wa** of the web toward the center of the rotation apparatus **1**. Alternatively, the directing part may be a mechanism for sucking the web **W** toward the center of the rotation apparatus **1** by vacuum suction. In the case where the directing part is a mechanism for jetting air, one or a plurality of directing parts may be provided. Furthermore, in the case where the directing part is a mechanism for pushing the loose portion **Wa**, one or a plurality of directing parts may be provided. By providing a plurality of directing parts, the loose portion **Wa** can be bent exactly toward the center of the rotation apparatus **1**. In the present embodiment, the loose portion **Wa** may have a shape dented toward the center of the rotation apparatus **1** due to the weight of the second web **W2**.

Next, a folding operation will be described. When the second web **W2** is sucked to be held on the surface of the pad **9₁** at the receiving position **RP**, the second web **W2** is transported along the pad **9₁** of the rotation apparatus **1**. When the pad **9₁** is rotated from the receiving position **RP** to the contact point O, the interval between the pads **9ᵢ** is decreased. Because of this, the loose portion **Wa** is formed in the second web **W2**. Furthermore, the loose portion **Wa** is folded into two to form a folded portion **Wb.**

The rotation apparatus for conducting the above-mentioned "folding" is not limited to the rotation apparatus **1**. For example, an apparatus described in Japanese Patent Application No. 2001-545183 A may be used. The present invention is intended to place an elastic member intermittently on a web. Therefore, the second web **W2** is not necessarily folded completely. Furthermore, depending upon the shape of the pad **9ᵢ**, the second web **W2** may have a loop shape.

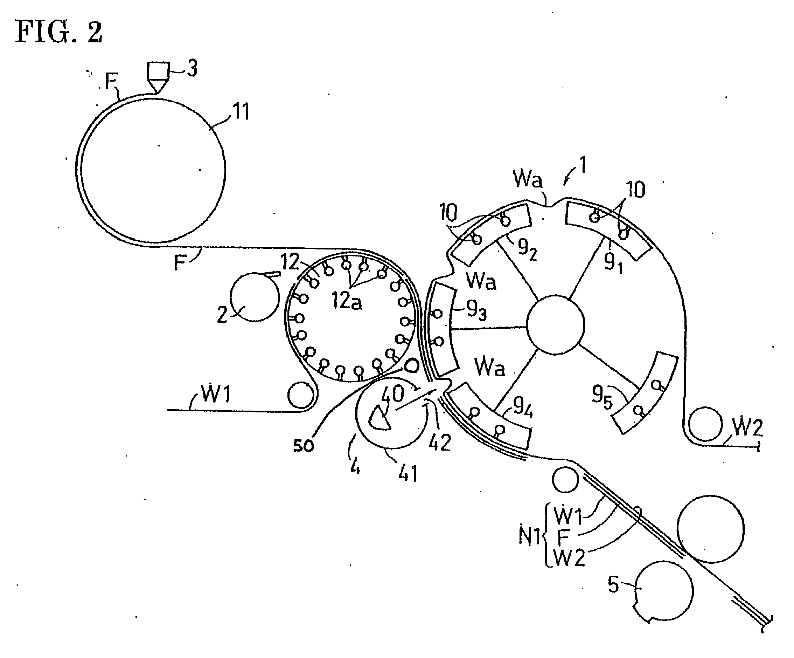

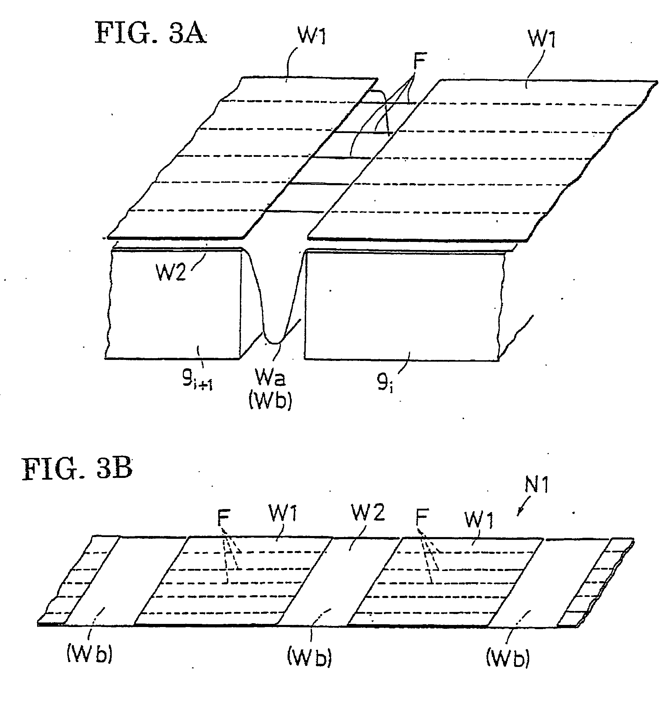

The folded second web **W2** is attached to be stacked on the first web **W1** at non-folded portions **Wc** before and after the folded portion **Wb.** That is, the elastic member **F** is not cut until the elastic member **F** is placed and fixed between the first web **W1** and the second web **W2.** Therefore, the stretched elastic member **F** hardly shrinks on the second web **W2.** When the second web **W2** is stacked on the first web **W1**, the first web **W1** is not placed on the folded portion **Wb** folded between the pads **9ᵢ** and **9ᵢ₊₁** adjacent to each other in the vicinity of the contact point **O**. When the interval between the pads **9ᵢ** and **9ᵢ₊₁** is increased as shown in FIG. **2**, only the elastic member **F** is exposed as shown in FIG. **3A**.

As shown in FIG. **1**, during β from a time when the first web **W1** is attached to the second web **W2** to a time when the first web **W1** is received, suction of air through the suction holes **12a** of the second roller **12** may be stopped or air may be discharged through the suction holes **12a**. The purpose of this is to attach the first web **W1** to the second web **W2** smoothly.

The exposed elastic member **F** in FIG. **3A** is cut with a cutter. In the case where the elastic member **F** can be cut with a light cutter, a light cutter **4** shown in FIG. **2** is used. The light cutter **4** has a rotatable cover, and a slit **42** is formed at a part of the cover. The cover **41** with the slit **42** is rotated, whereby infrared rays are radiated intermittently from a light source **40** to the elastic member **F** to cut it at a predetermined pitch. When the elastic member **F** is cut with the light cutter **4**, there is no possibility that the webs **W1** and **W2** are damaged by heat from the light cutter **4**, since the elastic member **F** is away from the loose portion **Wa.** The elastic member **F** may be cut with a pressure, heat, UV-rays, a laser or at least two of them. Furthermore, the elastic member **F** may be cut with an ultrasonic wave.

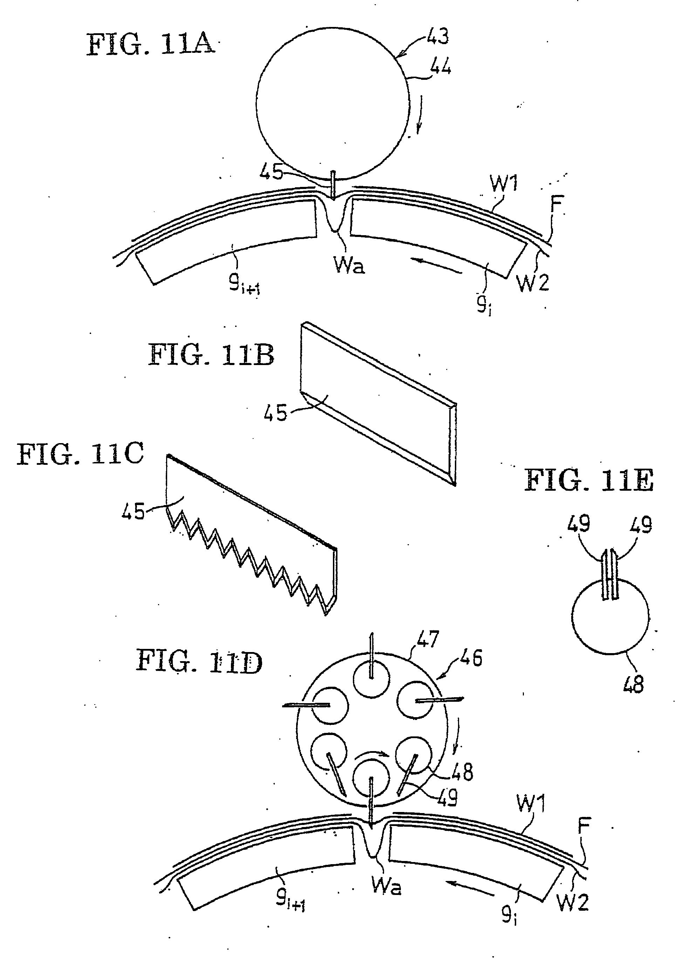

Hereinafter, an example of cutting the elastic member **F** with a cutter will be described. FIG. **11A** is a view showing a cutting machine **43** that is an example of a cutter. The cutting machine **43** has a rotatable roller **44** and at least one of blade **45** placed at the roller **44.** The distance between the rotation center of the roller **44** and the center of the rotation apparatus **1** is hardly changed. The blade **45** can cut the elastic member **F** positioned on the loose portion **Wa** between the pads **9ᵢ** and the pad **9ᵢ₊₁**, when the roller **44** is rotated. The rotation of the roller **44** is changed in accordance with the rotation of at least one of the pad **9ᵢ** and the pad **9ᵢ₊₁.** The blade (s) **45** may have a linear shape (FIG. **11B**) or may be in a jagged shape (FIG. **11C**). Furthermore, the blade **45** may be reciprocated in the direction of the rotation axis.

FIG. **11D** shows another cutting machine **46**. The cutting machine **46** includes a rotatable large roller **47**, a rotatable small roller **48** attached to the large roller **47**, and a blade **49** placed at the small roller **48.** The blade **49** of the cutting machine **46** can cut the elastic member F positioned on the loose portion **Wa** between the pad **9ᵢ** and the pad **9ᵢ₊₁**, when the large roller **47** and the small roller **48** are rotated. The rotations of the large roller **47** and the small roller **48** are changed in accordance with the rotation of at least one of the pad **9ᵢ** and the pad **9ᵢ₊₁**. As shown in FIG. **11D**, when the blade **49** reaches the bottom dead point, the tip end of the blade **49** is preferably directed to the center of the rotation apparatus **1**. Furthermore, in the same way as in the blade **45**, the blade **49** may have a shape as shown in FIG. **11B** or **11C**, and may be reciprocated in the direction of the rotation axis of the large roller **47** or the small roller **48**.

The small roller **48** of the cutting machine **46** may have a plurality of blades **49** as shown in FIG. **11E****.** The blades **49** may be reciprocated in the direction of the rotation axis to cut the elastic member.

The rotation apparatus **1** may be provided with scissors instead of the cutting machine. After the second web **W2** is stacked on the first web **W1**, and the interval between the pad **9ᵢ** and the pad **9ᵢ₊₁** is slightly increased, the scissors may enter between the pad **9ᵢ** and the pad **9ᵢ₊₁** to cut the elastic member stretched between the pad **9ᵢ** and the pad **9ᵢ₊₁**. The scissors may enter between the pad **9ᵢ** and the pad **9ᵢ₊₁** in accordance with the rotation of at least one of the pad **9ᵢ** and the pad **9ᵢ₊₁.** In order for the scissors to perform such an operation, at least one of a cam mechanism and a link mechanism may be used.

In FIG. **1**, the circumferential velocity of the pad **9₃** becomes a velocity **V31** (**V31** > **V21**) at a releasing position **SP** where the pad **9₃** releases the second web **W2.** Therefore, the interval between the pads **9₃** and **9₄** is increased from the contact point **O** to the releasing position **SP**. Therefore, the tension stress and elongation of the elastic member **F** are increased from the contact point **O** to the releasing position **SP.** When the elastic member **F** is cut with the light cutter **4** in this state, cutting becomes easy. At the releasing position **SP**, a stack **N1** in FIG. **3B**, in which the webs **W1** and **W2** and the elastic member **F** are stacked, is in the state before the folded portion **Wb** is folded.

Herein, during α from the releasing position **SP** to the receiving position **RP**, suction through the suction holes **10** may be stopped, and air may be discharged through the suction holes **10.** The purpose of this is to smoothly transfer the stack **N1** in a downstream direction.

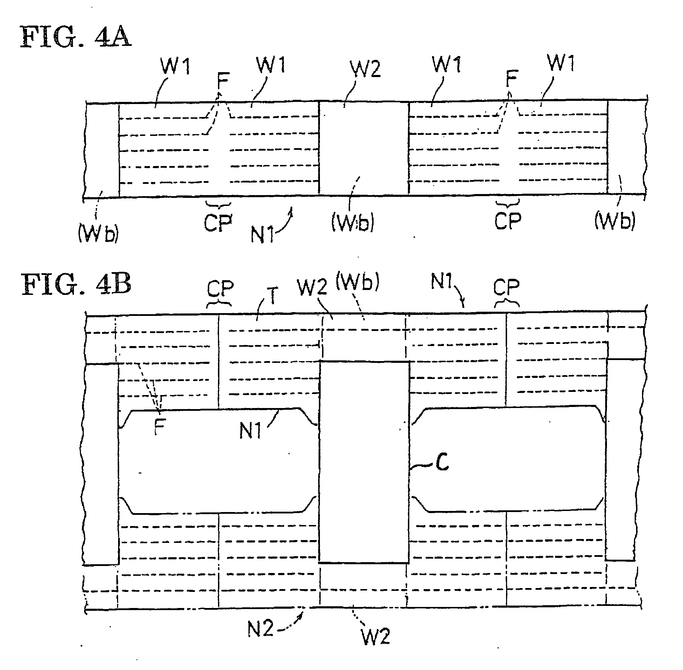

As shown in FIG. **1**, an embossing roller **5** may be provided downstream from the releasing position **SP.** The stack **N1** in FIG. **3B** is sent to the embossing roller **5**. A plurality of convex portions **51** are arranged on the embossing roller **5**, and at least the elastic member **F** of the stack **N1** is cut with the convex portions **51**. Because of this, as shown in FIG. **4A**, the stack **N1** is obtained in which the shrinking force of a part of the elastic member **F** interposed between the first and second webs **W1** and **W2** is suppressed. An adhesive is not basically applied to a portion **CP** where the shrinking force of the elastic member **F** is suppressed by the embossing roller **5**. The reason for this is to protect the shrinking of the elastic member **F**. However, in order to control the shrinking of the elastic member **F**, some adhesive weaker than usual may be applied.

A portion represented by a solid line in FIG. **4B** shows an example of a disposable diaper produced by the production method of the present invention. In the stack **N1**, at least a part of an absorber **C** is fixed at the portion **Wb** of the stack **N1** where the elastic member **F** is not placed. The other end of the absorber **C** is connected to another stack **N2** represented by a broken line. The absorber **C** is folded into two, and the portion **CP** of the stack **N1** comes into contact with a corresponding portion of another stack **N2**, whereby continuous disposable diapers can be produced. Another stack **N2** may have the same configuration as that of the stack **N1**. Furthermore, in the case where the absorber **C** is folded into two, and the portion **CP** of the stack **N1** comes into contact with the corresponding portion of another stack **N2**, whereby the stacks **N1** and **N2** are stretched in a flow direction, even if the elastic member **F** is placed at the portion **CP**, the above connection can be conducted.

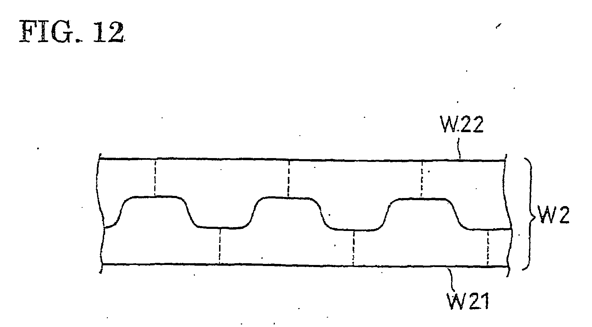

Furthermore, the following may also be possible: when the elastic member **F** is cut so that a trim is not generated as shown in FIG. **12**, and the phase of one web thus cut is shifted by substantially ½ from the other web, whereby the web **W2** shown in FIG. **4B** is formed. As a method for shifting a phase, one web may be allowed to pass through a dummy roller to cause a delay from the other web.

### Embodiment 2

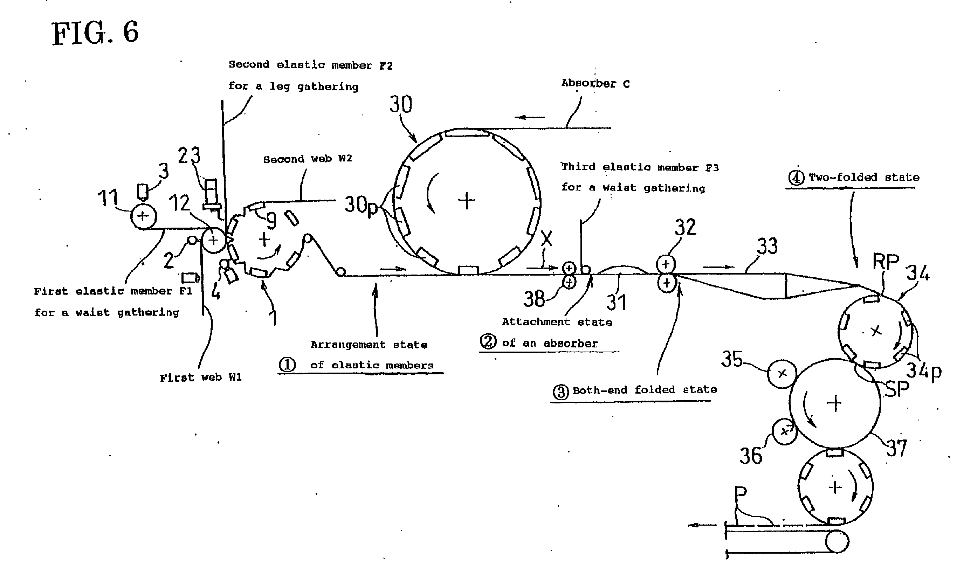

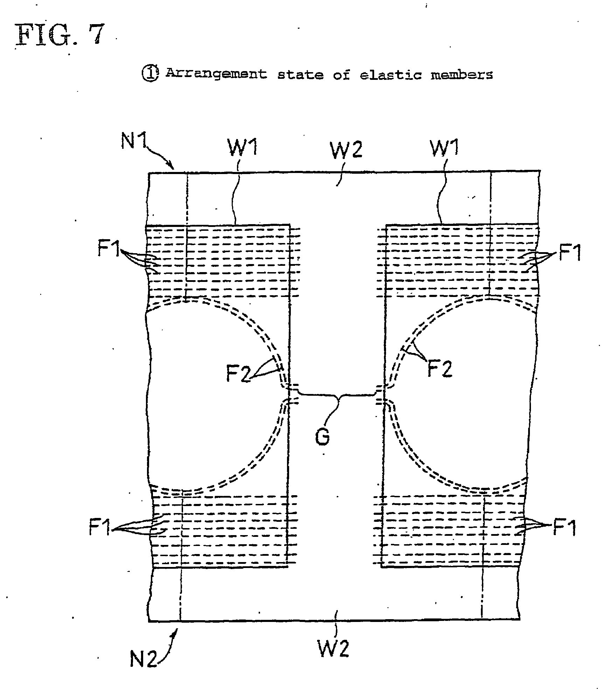

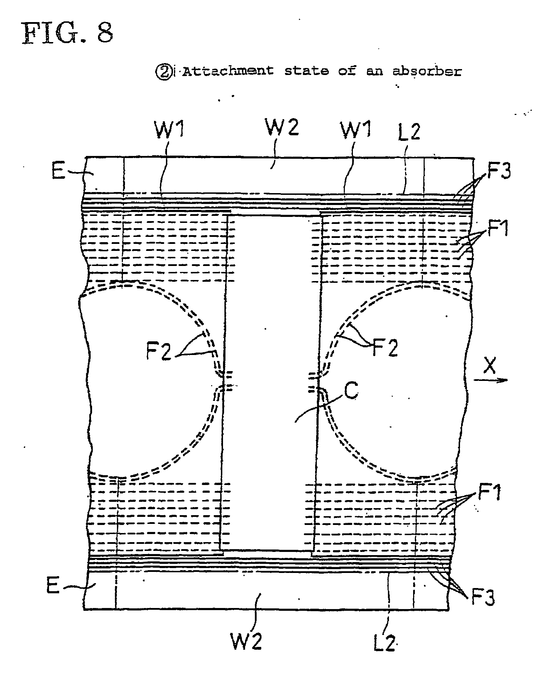

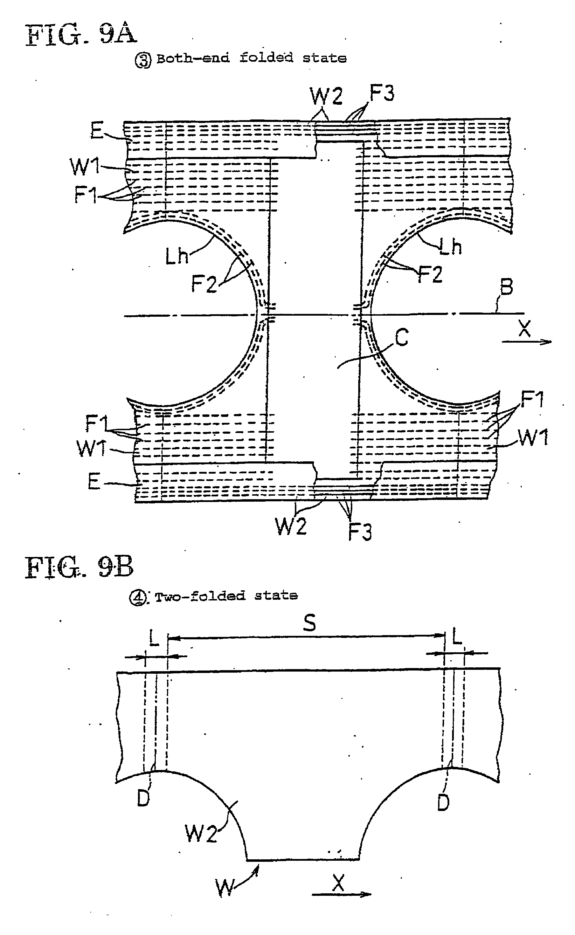

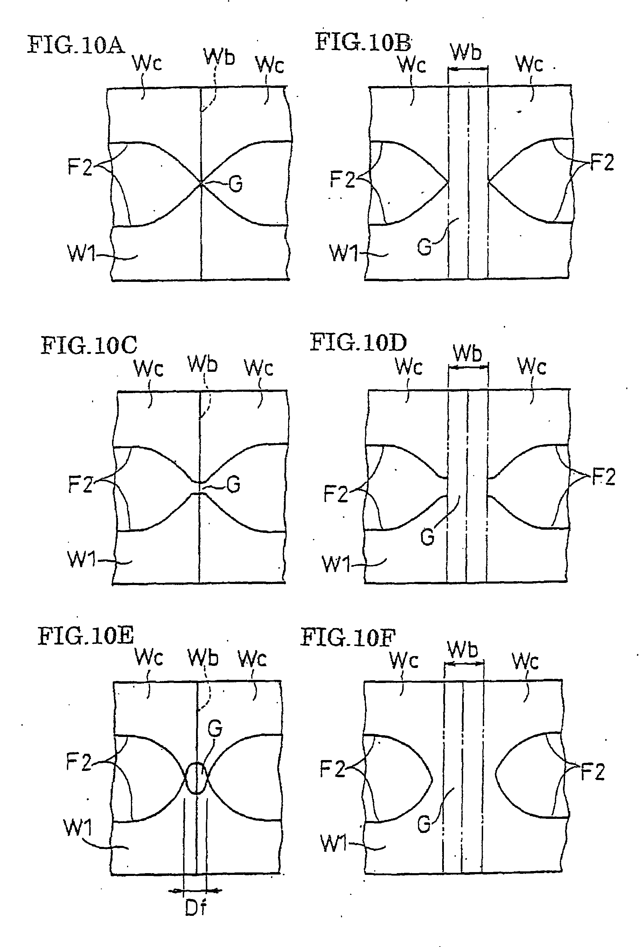

Next, Embodiment 2 will be described. A production apparatus shown in FIG. **6** is capable of placing at least three kinds of elastic members **F1** to **F3** for a leg gathering on a wearing article, as shown in FIG. **9A****.**
(1) Arrangement state of elastic members:
   First, as shown in FIG. **7**, the configuration and operation of the present apparatus for obtaining an arrangement state of elastic members, in which first and second elastic members **F1** and **F2** are placed on webs **W1** and **W2**, will be briefly described.
   The present apparatus includes a first roller **11** for placing a first elastic member **1** for a waist gathering and a guide unit **23** for placing a second elastic member **2** for a leg gathering. The guide unit **23** is capable of arranging the second elastic member **F2** for a leg gathering in a curved shape or a linear shape by being reciprocated in the axis direction of a second roller **12** with, for example, a cam or a servo motor. U.S. Patent Application No. 2003/001042 discloses an example of the guide unit **23.** The second elastic member **F2** may be cut mechanically with scissors, a blade, or the like provided at the roller. FIGS **11A** to **11D** show examples of a cutter. The first elastic member **F1** may not be a thermoplastic elastic member. In this case, the first elastic member **F1** can be cut with scissors, a blade, or the like in the same way as in the second elastic member **F2.** Furthermore, the first and second elastic members **F1** and **F2** may be cut with the same scissors, blade, or the like. In the steps until here in Embodiment 2, the other configuration is the same as that of Embodiment 1. Like parts are denoted with like reference numerals, and the detailed description and drawings are omitted here.
   After being interposed and fixed between the first and second webs **W1** and **W2**, the first and second elastic members **F1** and **F2** are cut to be arranged as shown in FIG. **7**.
   In some disposable wearing articles, an elastic member for a leg gathering is placed on a crotch portion. Therefore, such a portion contracts to cause an unsatisfactory feeling during wearing. In order to avoid this, the elastic member on the crotch portion is cut so as to shrink, whereby the elastic member on the crotch portion can be made short to some degree. However, it is impossible to completely eliminate the elastic member on the crotch portion, so that an unsatisfactory feeling may still remain.
   In contrast, when the second elastic member **F2** for a leg gathering is placed as shown in FIGS. **10A**, **10C**, and **10E**, the second elastic member **F2** can be eliminated from the crotch portion G. FIGS. **10A**, **10C**, and **10E** show the second elastic member **F2** interposed between the first web **W1** and the second web **W2** on adjacent pads **9**, wherein a part of the web **W2** is interposed between the adjacent pads **9**.
   For example, as shown in FIGS. **10A** and **10c**, a folded portion **Wb** is formed on the crotch portion **G** of the first web **W1**, and the second elastic member **F2** is placed on non-folded portions **Wc**. Thereafter, the second elastic member **F2** striding across the adjacent pads **9** is cut. As a result, the second elastic member **F2** is not placed on the crotch portion **G**, as shown in FIGS. **10B** and **10D****.**
   Furthermore, as shown in FIG. **10e**, when a non-coated portion **Df** with no adhesive applied thereto is provided in the non-folded portion **Wc** adjacent to the vicinity of the folded portion **Wb**, after the second elastic member **F2** is cut, a portion of the second elastic member **F2** that is not sufficiently attached to the non-folded portion **Wc** shrinks (FIG. **10F**). Full-circle pants may be produced by such adjustment of an adhesive. Some adhesive may be applied to the non-coated portion **Df**. In this case, the second elastic member **F2** shrinks slowly without being fixed to the non-folded portion **Wc**, and finally, the second elastic member **F2** can be eliminated from the crotch portion **G**.
   The adhesive only needs to be applied to a portion where the second elastic member **F2** is placed. As a coating method, an adhesive may be applied in an annular shape to at least one of the first and second webs **W1** and **W2** with a coater or the like. Furthermore, guns with a plurality of valves are placed in a direction across the first web **W1** so that opening/closing of the valve may be controlled by the flow speed of at least one of the first web **W1** and the second web **W2** and the coating shape. In the case of using such guns, an adhesive can be applied in arbitrary regions partitioned in a matrix.
(2) Attachment state of an absorber:
   An absorber **C** is arranged and attached to the webs **W1** and **W2** in the above-mentioned arrangement state of the elastic members by a turn apparatus **30**. Thereafter, a third elastic member **F3** for a waist gathering is placed to form an attachment state of an absorber as shown in FIG. **8**.
   The turn apparatus **30** is provided with a plurality of suction pads **30p** around a drum (not shown) . The suction pads **30p** are rotated in an arrow direction while changing the posture of the absorber **C** by 90° by turning. As the turn apparatus **30**, for example, an apparatus as disclosed by International Publication No. WO 01/44086 or U.S. Patent Application No. US 2002/0103468 may be used.
(3) Both-edge folded state:
   Thereafter, the webs **W1** and **W2** with the absorber attached thereto have their folding portions **E** on both edges folded inward at a position of two-dot dash lines **L₂** shown in FIG. **8** by a folding apparatus **31**. After both edges of the webs **W1** and **W2** are folded, a leg hole Lh is cut out with a leg hole cutter **32** (FIG. **6**), whereby a both-edge folded state shown in FIG. **9A** is formed. The leg hole cutter **32** may be placed upstream from the turn apparatus **30**. In such a configuration, it also becomes possible to produce a disposable wearing article in which a part of the absorber **C** extends out to the leg hole **Lh** portion.
(4) Two-folded state:
   After folding, the webs **W1** and **W2** are folded into two at a folding line **B** by a two-folding apparatus **33**, whereby the webs **W1** and **W2** are folded into two as shown in FIG. **9B**. As the two-folding apparatus **33**, for example, an apparatus described in U.S. Patent No. 3,828,367, U.S. Patent No. 5,711,832, or U.S. Patent No. 6913664 may be used.
   Thereafter, the webs **W1** and **W2** folded into two have both edges L sealed (e.g., heat-sealed or sonic-sealed), as shown in FIG. **9B**. Thereafter, a cutting portion **D** represented by a two-dot dash line is cut to form an individual disposable wearing article, e.g., pants **P**.
   The present apparatus may form pants **P** from the webs **W1** and **W2** (hereinafter, referred to as a "stacked web **W**") folded into two, using a decelerating drum **34**, a main drum **37**, a seal roller **35**, and a cutter roller **36**.

### Decelerating drum 34:

As described above, although the web **W** has an elastic member, the web **W** is moved in a stretched state so that placement of an absorber at a predetermined position, formation of a leg hole, and other processing can be performed easily. However, in order to seal the web **W** and cut a seal portion, the position of the edge L only needs to be determined. This is because the state of the other portions of the web **W** does not influence a sealing step and a cutting step. Furthermore, in the sealing step, it is preferable that a flow speed of the web **W** is lower so as to obtain a sufficient time for melting a part of the web **W** and the like. The decelerating drum can decrease the flow speed of the web **W** so as to obtain a sufficient time for melting the part of the web **W** and the like. As the decelerating drum **34**, an apparatus described in JP 63-317576 A, International Publication No. WO 01/44086, or U.S. Patent Application No. 2002/0103468 may be used.

The decelerating drum **34** is provided with a plurality of pads **34p** rotating in a transport direction of the stacked web **W** around a drum (not shown) . The decelerating drum **34** receives at least an edge **L** at a receiving position RP from the two-folding apparatus **33** placed upstream therefrom, and holds it. The other portions of the web **W** are positioned between adjacent pads **34p**. The edge **L** is transported to a releasing position **SP**, and thereafter, the edge **L** is supplied to the main drum **37** placed downstream therefrom at the releasing position **SP**. While the pad **34p** moves from the receiving position **RP** to the releasing position **SP**, the interval between the adjacent pads **34p** is changed to be narrow.

The pad **34p** is capable of receiving the edge **L** in a stretched state at the receiving position **RP**, and keeping holding the edge **L** shown in FIG. **9B**. For example, the edge **L** may be held by sucking the edge **L**, pressing the edge **L** with a hook, or fixing the edge **L** with a pin placed at the pad **34p**. On the other hand, the interval between the adjacent pads **34p**, which has been increased at the receiving position **RP**, is decreased before the releasing position **SP**, and a shrinking portion **S** excluding the edge **L** shrinks in a transport direction **X**. In order to allow the edge **L** to be exactly held, the shrinking force of the elastic member positioned at the edge **L** may be weakened by an embossing roller **38**. For example, the embossing roller **38** can cut the elastic member to a predetermined length or change the elastic configuration of the elastic member by using at least one of heat and pressure. As the embossing roller **38**, for example, an apparatus described in U.S. Patent Application No. 2002/0103468 may be used. The embossing roller **38** is placed in a region from the rotation apparatus **1** to the two-folding apparatus **33.**

### Main drum 37:

The main drum **37** receives the web **W** in a mixed state where the stretched edge **L** and the shrinking portion **S** that has shrunk are present alternately from the decelerating drum **34.** The main drum **37** transports the web **W** in such a mixed state. In order to keep the mixed state, the main drum **37** may be provided with, for example, a plurality of suction holes to suck air through the suction holes so as to suck the web **W**.

### Seal roller 35:

The seal roller **35** can conduct heat-sealing by heating and pressing the edge **L** (FIG. **9B**) of the web **W**. More specifically, the web **W** is pressed by being interposed between the seal roller **35** and the main drum **37**, and heated by the seal roller **35**. The seal roller **35** may be provided with, for example, a heater so as to heat-seal the edge **L**. Furthermore, the seal roller **35** may have a horn to conduct sonic-sealing. The web **W** subjected to the above-mentioned heat-sealing is cut to individual pants **P** by a cutter roller **36** placed downstream therefrom.

### Formation operation of pants P:

Next, an operation of forming pants **P** from the stacked web **W** will be described. The decelerating drum **34** continuously receives the web **W** onto each pad **34p** under the condition that the web **W** is stretched in a flow direction. Herein, the pad **34p** holds the edge **L** (FIG. **9B**) of the web **W** in a stretched state.

After receiving the web **W**, the pad **34p** having received the web **W** has its circumferential velocity decreased, whereby the interval between the adjacent pads **34p** becomes narrow. Therefore, a portion **S** other than the edge **L** of the web **W** shown in FIG. **9B** shrinks in a transport direction **X** to form the shrinking portion **S**. On the other hand, the edge **L** is held in a stretched state.

Thereafter, the main drum **37** receives the web **W** from the decelerating drum **34**. At this time, the main drum **37** receives the web **W** in a mixed state where the edge **L** in a stretched state and the shrinking portion **S** that has shrunk are present alternately. The main drum **37** transports the web **W** in a downstream direction while keeping the mixed state. During transportation by the main drum **37**, the seal roller **35** presses and heats the edge **L** of the web **W** on the main drum **37**. Herein, a part of the web **W** shrinks, so that the transport velocity of the web becomes low. Sealing can be conducted exactly, as the transport velocity of the web **W** is low.

Thereafter, the web **W** is cut to individual pieces with a cutter roller **36** to form pants **P**.

As described above, according to the present invention, after being interposed and fixed between a first web and a second web, an elastic member is cut. Therefore, the stretched elastic member hardly shrinks on the second web, whereby an expected gathering is obtained.

Furthermore, according to the present invention, the elastic member is cut between adjacent first webs. Therefore, the second web and an absorber do not become stiff between the first webs.

Furthermore, a plurality of first webs are placed on a second web under the condition where they are away from each other. Therefore, the first web is not stacked on a portion where a gathering of the elastic member is not formed, so that such a portion becomes thin and allows air to pass therethrough. As a result, a user wearing such an article is not likely to feel stuffy.

Furthermore, the elastic member is cut between the first webs and at a loose portion of the second web. Therefore, only the elastic member can be cut without damaging the web.

If a pad that makes the second web loose is allowed to transport the second web at a substantially constant velocity during a predetermined period from a time when the pad starts receiving the first web to a time when the pad finishes receiving it, the first web can be transferred to a predetermined position of the second web exactly.

Furthermore, if the elastic member is cut under the condition that the tension stress of the elastic member is increased, the elastic member can be cut easily with infrared rays or the like.

Furthermore, while the web is transported under the condition that an edge (where the web is stretched) and a shrinking portion (where the web shrinks) are present alternately in a transport direction, a heating time for heating the edge becomes long. Therefore, the transport velocity of the web can be increased without impairing the exactness of heat-sealing.

Various other modifications will be apparent to and can be readily made by those skilled in the art without departing from the scope and spirit of this invention. Accordingly, it is not intended that the scope of the claims appended hereto be limited to the description as set forth herein, but rather that the claims be broadly construed.

## Claims

1. A method of producing a wearing article, the method comprising the steps of:
supplying an elastic member (F, F1, F2);
stretching the elastic member (F, F1, F2);
placing the stretched elastic member (F, F1, F2) so that the elastic member (F, F1, F2) spreads across a plurality of first webs (W1) divided in a transport direction;
making a part of a second web (W2) loose in the transport direction while transporting the second web (W2), thereby forming a loose portion (Wa, Wb);
placing the first webs (W1), on which the elastic member (F, F1, F2) is disposed, on non-loose portions (Wc) before and after the loose portion (Wa, Wb) of the second web (W2) such that the elastic member (F, F1, F2) is between the first webs (W1) and the second web (W2), but not placing the first webs (W1), on which the elastic member (F, F1, F2) is disposed, on the loose portion (Wa, Wb) of the second web (W2);
cutting the elastic member (F, F1, F2) between adjacent first webs (W1) of the plurality of first webs (W1); and
placing an absorber (C) on a region of the second web between adjacent first webs with the elastic member (F, F1, F2) cut therebetween.

2. A method of producing a wearing article according to claim 1, wherein, in the step of forming the loose portion (Wa, Wb), the second web is transported at a substantially constant velocity during a predetermined period by a first pad (9) among a plurality of pads (9) disposed away from each other and having varying movement velocities, and an interval between the first pad (9) and a preceding pad (9) adjacent to the first pad (9) is decreased to generate the loose portion (Wa, Wb).

3. The method for producing a wearing article according to claim 1, wherein the step of placing the first webs (W1) further comprises the step of, while pads (9) corresponding to non-loose portions (Wc) of the second web (W2) among the plurality of pads (9) disposed away from each other and having varying movement velocities are rotated at a substantially constant velocity during a predetermined period, placing the first webs (W1) on the second webs on the pads (9) rotated at the constant velocity; and increasing an interval between the first webs (W1) placed on the non-loose portions (Wc) by allowing an interval between the plurality of pads (9) after placement of the first webs (W1).

4. The method for producing a wearing article according to any one of claims 1 to 3, wherein a material containing, as the elastic member (F, F1, F2), thread rubber, flat rubber, mesh-shaped rubber, a film, or a thermoplastic elastic member (F, F1, F2).

5. The method for producing a wearing article according to any one of claims 1 to 4, wherein the elastic member (F, F1, F2) is cut by adding at least heat to the plastic member (F, F1, F2) between the adjacent first webs (W1).

6. The method for producing a wearing article according to claim 5, wherein the elastic member (F, F1, F2) is cut in a state where stress of the elastic member (F, F1, F2) is increased by allowing the adjacent first webs (W1) to be away from each other during transportation.

7. The method for producing a wearing article according to any one of claims 1 to 4, wherein the elastic member (F, F1, F2) between the adjacent first webs (W1) is cut with scissors.

8. An apparatus for producing a wearing article, the apparatus comprising:
means for supplying an elastic member (F, F1, F2);
means for stretching the elastic member (F, F1, F2);
means for placing the stretched elastic member (F, F1, F2) so that the elastic member (F, F1, F2) spreads across a plurality of first webs (W1) divided in a transport direction;
means for making a part of a second web (W2) loose in the transport direction while transporting the second web (W2), thereby forming a loose portion (Wa, Wb);
means for placing the first webs (W1), on which the elastic member (F, F1, F2) is disposed, on non-loose portions (Wc) before and after the loose portion (Wa, Wb) of the second web (W2) such that the elastic member (F, F1, F2) is between the first webs (W1) and the second web (W2), but not placing the first webs (W1), on which the elastic member (F, F1, F2) is disposed, on the loose portion (Wa, Wb) of the second web (W2);
a cutter (4) for cutting the elastic member (F, F1, F2) between adjacent first webs (W1) of the plurality of first webs (W1); and
means for placing an absorber (C) on a region of the second web between adjacent first webs with the elastic member (F, F1, F2) cut therebetween.

9. An apparatus according to claim 8, further comprising:
a roller (12) for supplying the plurality of first webs (W1) divided in a transport direction at a predetermined position in a state where the elastic member (F1, F1, F2) is placed so as to spread across the first webs (W1); and
a rotation apparatus (1) including a plurality of pads (9) which are rotated to continuously transport the second web (W2) and approach the roller (12) at the predetermined position, wherein the respective pads (9) of the rotation apparatus (1) have varying circumferential velocities,
wherein the respective pads (9) of the rotation apparatus (1) receive the second web (W2) at a receiving position upstream from the predetermined position, and after receiving, an interval between the adjacent pads (9) is decreased while the pads (9) are rotated to the predetermined position, whereby the loose portion is formed in the second web (W2),
wherein the first webs (W1) are placed to be stacked on a surface of the second web (W2) on a basis of the pads (9) at the predetermined position,
wherein an interval between the adjacent pads (9) is increased while the respective pads (9) of the rotation apparatus (1) are rotated from the predetermined position to a releasing position downstream from the predetermined position, and
wherein the cutter (4) cuts, in use, the elastic member (F, F1, F2) in the loose portion (Wa, Wb) while the pads (9) are rotated from the predetermined position to the releasing position.

## Patentansprüche

1. Verfahren zur Herstellung eines tragbaren Artikels, wobei das Verfahren die folgenden Schritte aufweist:
Zuführen eines elastischen Elementes (F, F1, F2);
Dehnen des elastischen Elementes (F, F1, F2);
Anordnen des gedehnten elastischen Elementes (F, F1, F2), so dass sich das elastische Element (F, F1, F2) über eine Vielzahl von ersten Bahnen (W1) ausbreitet, die in einer Transportrichtung getrennt sind;
Lockern eines Teils einer zweiten Bahn (W2) in der Transportrichtung, während die zweite Bahn (W2) transportiert wird, wodurch ein lockerer Abschnitt (Wa, Wb) gebildet wird;
Anordnen der ersten Bahnen (W1), auf denen das elastische Element (F, F1, F2) angeordnet ist, auf nicht lockeren Abschnitten (Wc) vor und nach dem lockeren Abschnitt (Wa, Wb) der zweiten Bahn (W2), so dass das elastische Element (F, F1, F2) zwischen den ersten Bahnen (W1) und der zweiten Bahn (W2) ist, aber nicht Anordnen der ersten Bahnen (W1), auf denen das elastische Element (F, F1, F2) angeordnet ist, auf dem lockeren Abschnitt (Wa, Wb) der zweiten Bahn (W2);
Schneiden des elastischen Elementes (F, F1, F2) zwischen benachbarten ersten Bahnen (W1) der Vielzahl der ersten Bahnen (W1); und
Anordnen eines absorbierenden Materials (C) auf einem Bereich der zweiten Bahn zwischen benachbarten ersten Bahnen, wobei das elastische Element (F, F1, F2) dazwischen geschnitten wird.

2. Verfahren zur Herstellung eines tragbaren Artikels nach Anspruch 1, bei dem beim Schritt des Bildens des lockeren Abschnittes (Wa, Wb) die zweite Bahn mit einer im Wesentlichen konstanten Geschwindigkeit während einer vorgegebenen Periode mittels eines ersten Kissens (9) unter einer Vielzahl von Kissen (9), die voneinander weg angeordnet sind und variierende Bewegungsgeschwindigkeiten aufweisen, transportiert wird, und wobei ein Intervall zwischen dem ersten Kissen (9) und einem vorhergehenden Kissen (9), benachbart dem ersten Kissen (9), verringert wird, um den lockeren Abschnitt (Wa, Wb) zu erzeugen.

3. Verfahren zur Herstellung eines tragbaren Artikels nach Anspruch 1, bei dem der Schritt des Anordnens der ersten Bahnen (W1) außerdem die folgenden Schritte aufweist: während die Kissen (9), die nicht lockeren Abschnitten (Wc) der zweiten Bahn (W2) unter der Vielzahl der Kissen (9), die voneinander weg angeordnet sind und variierende Bewegungsgeschwindigkeiten aufweisen, entsprechen, mit einer im Wesentlichen konstanten Geschwindigkeit während einer vorgegebenen Periode gedreht werden, das Anordnen der ersten Bahnen (W1) auf den zweiten Bahnen auf den Kissen (9), die mit der konstanten Geschwindigkeit gedreht werden; und das Vergrößern eines Intervalls zwischen den ersten Bahnen (W1), die auf den nicht lockeren Abschnitten (Wc) angeordnet sind, indem ein Intervall zwischen der Vielzahl der Kissen (9) nach der Anordnung der ersten Bahnen (W1) gestattet wird.

4. Verfahren zur Herstellung eines tragbaren Artikels nach einem der Ansprüche 1 bis 3, bei dem ein Material als das elastische Element (F, F1, F2) einen Fadengummi, einen flachen Gummi, einen maschenartigen Gummi, eine Folie oder ein thermoplastisches elastisches Element (F, F1, F2) enthält.

5. Verfahren zur Herstellung eines tragbaren Artikels nach einem der Ansprüche 1 bis 4, bei dem das elastische Element (F, F1, F2) geschnitten wird, indem dem elastischen Element (F, F1, F2) zwischen den benachbarten ersten Bahnen (W1) mindestens Wärme hinzugefügt wird.

6. Verfahren zur Herstellung eines tragbaren Artikels nach Anspruch 5, bei dem das elastische Element (F, F1, F2) in einem Zustand geschnitten wird, wo die Belastung des elastischen Elementes (F, F1, F2) vergrößert wird, indem zugelassen wird, dass die benachbarten ersten Bahnen (W1) während des Transportes voneinander weg sind.

7. Verfahren zur Herstellung eines tragbaren Artikels nach einem der Ansprüche 1 bis 4, bei dem das elastische Element (F, F1, F2) zwischen den benachbarten ersten Bahnen (W1) mit Scheren geschnitten wird.

8. Vorrichtung zur Herstellung eines tragbaren Artikels, wobei die Vorrichtung aufweist:
ein Mittel für das Zuführen eines elastischen Elementes (F, F1, F2);
ein Mittel für das Dehnen des elastischen Elementes (F, F1, F2);
ein Mittel für das Anordnen des gedehnten elastischen Elementes (F, F1, F2), so dass sich das elastische Element (F, F1, F2) über eine Vielzahl von ersten Bahnen (W1) ausbreitet, die in der Transportrichtung getrennt sind;
ein Mittel für das Lockern eines Teils einer zweiten Bahn (W2) in der Transportrichtung, während die zweite Bahn (W2) transportiert wird, wodurch ein lockerer Abschnitt (Wa, Wb) gebildet wird;
ein Mittel für das Anordnen der ersten Bahnen (W1), auf denen das elastische Element (F, F1, F2) angeordnet ist, auf den nicht lockeren Abschnitten (Wc) vor und nach dem lockeren Abschnitt (Wa, Wb) der zweiten Bahn (W2), so dass das elastische Element (F, F1, F2) zwischen den ersten Bahnen (W1) und der zweiten Bahn (W2) ist, aber nicht Anordnen der ersten Bahnen (W1), auf denen das elastische Element (F, F1, F2) angeordnet ist, auf dem lockeren Abschnitt (Wa, Wb) der zweiten Bahn (W2);
ein Schneidemittel (4) für das Schneiden des elastischen Elementes (F, F1, F2) zwischen benachbarten ersten Bahnen (W1) der Vielzahl der ersten Bahnen (W1); und
ein Mittel für das Anordnen eines absorbierenden Materials (C) auf einem Bereich der zweiten Bahn zwischen benachbarten ersten Bahnen, wobei das elastische Element (F, F1, F2) dazwischen geschnitten wird.

9. Vorrichtung nach Anspruch 8, die außerdem aufweist:
eine Rolle (12) für das Zuführen der Vielzahl der ersten Bahnen (W1), getrennt in einer Transportrichtung in einer vorgegebenen Position in einem Zustand, wo das elastische Element (F, F1, F2) so angeordnet wird, dass es sich über die ersten Bahnen (W1) ausbreitet; und
eine Rotationsvorrichtung (1), die eine Vielzahl von Kissen (9) umfasst, die gedreht werden, um die zweite Bahn (W2) kontinuierlich zu transportieren und sich der Rolle (12) in der vorgegebenen Position zu nähern, wobei die jeweiligen Kissen (9) der Rotationsvorrichtung (1) variierende Umfangsgeschwindigkeiten aufweisen,
wobei die jeweiligen Kissen (9) der Rotationsvorrichtung (1) die zweite Bahn (W2) in einer Aufnahmeposition stromaufwärts von der vorgegebenen Position aufnehmen und nach dem Aufnehmen ein Intervall zwischen den benachbarten Kissen (9) verkleinert wird, während die Kissen (9) in die vorgegebene Position gedreht werden, wodurch der lockere Abschnitt in der zweiten Bahn (W2) gebildet wird,
wobei die ersten Bahnen (W1) so angeordnet werden, dass sie auf einer Oberfläche der zweiten Bahn (W2) auf einer Basis der Kissen (9) in der vorgegebenen Position aufgeschichtet werden,
wobei ein Intervall zwischen den benachbarten Kissen (9) vergrößert wird, während die jeweiligen Kissen (9) der Rotationsvorrichtung (1) aus der vorgegebenen Position in eine Freigabeposition stromabwärts von der vorgegebenen Position gedreht werden, und
wobei das Schneidemittel (4) bei Benutzung das elastische Element (F, F1, F2) im lockeren Abschnitt (Wa, Wb) schneidet, während die Kissen (9) aus der vorgegebenen Position in die Freigabeposition gedreht werden.

## Revendications

1. Procédé de production d'un article vestimentaire, le procédé comprenant les étapes ci-dessous :
amenée d'un élément élastique (F, F1, F2) ;
étirement de l'élément élastique (F, F1, F2) ;
mise en place de l'élément élastique étiré (F, F1, F2) de sorte que l'élément élastique (F, F1, F2) s'étend à travers plusieurs premières bandes (W1) divisées dans une direction de transport ;
desserrement d'une partie d'une deuxième bande (W2) dans la direction de transport pendant le transport de la deuxième bande (W2), pour former ainsi une partie desserrée (Wa, Wb) ;
mise en place des premières bandes (W1), sur lesquelles l'élément élastique (F, F1, F2) est agencé, sur des parties non desserrées (Wc), devant et derrière la partie desserrée (Wa, Wb) de la deuxième bande (W2), de sorte que l'élément élastique (F, F1, F2) se situe entre les premières bandes (W1) et la deuxième bande (W2), sans mise en place des premières bandes (W1) sur lesquelles est agencé l'élément élastique (F, F1, F2), sur la partie desserrée (Wa, Wb) de la deuxième bande (W2) ;
coupe de l'élément élastique (F, F1, F2) entre des premières bandes adjacentes (W1) des plusieurs premières bandes (W1) ; et
mise en place d'un absorbeur (C) sur une région de la deuxième bande entre des premières bandes adjacentes, l'élément élastique (F, F1, F2) étant coupé entre elles.

2. Procédé de production d'un article vestimentaire selon la revendication 1, dans lequel, lors de l'étape de formation de la partie desserrée (Wa, Wb), la deuxième bande est transportée à une vitesse essentiellement constante au cours d'une période prédéterminée par un premier patin (9) de plusieurs patins (9), agencés à l'écart les uns des autres et présentant des vitesses de déplacement variables, un intervalle entre le premier patin (9) et un patin précédent (9) adjacent au premier patin (9) étant réduit pour produire la partie desserrée (Wa, Wb).

3. Procédé de production d'un article vestimentaire selon la revendication 1, dans lequel l'étape de mise en place des premières bandes (W1) comprend en outre les étapes, consistant, pendant que les patins (9) correspondant à des parties non desserrées (Wc) de la deuxième bande (W2), parmi les plusieurs patins (9) agencés à l'écart les uns des autres et présentant des vitesses de déplacement variables sont tournées à une vitesse essentiellement constante pendant une période prédéterminée, à mettre en place les premières bandes (W1) sur les deuxièmes bandes sur les patins (9) tournés à la vitesse constante ; et à accroître un intervalle entre les premières bandes (W1) placées sur les parties non desserrées (Wc) en permettant un intervalle entre les plusieurs patins (9) après la mise en place des premières bandes (W1).

4. Procédé de production d'un article vestimentaire selon l'une quelconque des revendications 1 à 3, dans lequel un matériau contient, comme élément élastique (F, F1, F2), du caoutchouc en fils, du caoutchouc plat, du caoutchouc en forme de mailles, un film, ou un élément élastique thermoplastique (F, F1, F2).

5. Procédé de production d'un article vestimentaire selon l'une quelconque des revendications 1 à 4, dans lequel l'élément élastique (F, F1, F2) est coupé en appliquant au moins de la chaleur à l'élément élastique (F, F1, F2) entre les premières bandes adjacentes (W1).

6. Procédé de production d'un article vestimentaire selon la revendication 5, dans lequel l'élément élastique (F, F1, F2) est coupé dans un état dans lequel la contrainte de l'élément élastique (F, F1, F2) est accrue en permettant un écartement mutuel des premières bandes adjacentes (W1) au cours du transport.

7. Procédé de production d'un article vestimentaire selon l'une quelconque des revendications 1 à 4, dans lequel l'élément élastique (F, F1, F2) entre les premières bandes adjacentes (W1) est coupé par des ciseaux.

8. Appareil de production d'un article vestimentaire, l'appareil comprenant :
un moyen pour amener un élément élastique (F, F1, F2) ;
un moyen pour étirer l'élément élastique (F, F1, F2) ;
un moyen pour mettre en place l'élément élastique étiré (F, F1, F2) de sorte que l'élément élastique (F, F1, F2) s'étend à travers plusieurs premières bandes (W1) divisées dans une direction de transport ;
un moyen pour desserrer une partie d'une deuxième bande (W2) dans la direction du transport pendant le transport de la deuxième bande (W2), pour former ainsi une partie desserrée (Wa, Wb) ;
un moyen pour mettre en place les premières bandes (W1), sur lesquelles l'élément élastique (F, F1, F2) est agencé, sur des parties non desserrées (Wc) devant et derrière la partie desserrée (Wa, Wb) de la deuxième bande (W2), de sorte que l'élément élastique (F, F1, F2) se situe entre les premières bandes (W1) et la deuxième bande (W2), sans mettre en place les premières bandes (W1), sur lesquelles l'élément élastique (F, F1, F2) est agencé, sur la partie desserrée (Wa, Wb) de la deuxième bande (W2) ;
un moyen de coupe (4) pour couper l'élément élastique (F, F1, F2) entre des premières bandes adjacentes (W1) des plusieurs premières bandes (W1) ; et
un moyen pour mettre en place un absorbeur (C) sur une région de la deuxième bande, entre des premières bandes adjacentes, l'élément élastique (F, F1, F2) étant coupé entre elles.

9. Appareil selon la revendication 8, comprenant en outre :
un rouleau (12) pour amener les plusieurs premières bandes (W1) divisées dans une direction de transport au niveau d'une position prédéterminée, dans un état dans lequel l'élément élastique (F, F1, F2) est mis en place de sorte à l'étirer à travers les premières bandes (W1) ; et
un dispositif de rotation (1), englobant plusieurs patins (9) qui sont tournés en vue de transporter en continu la deuxième bande (W2) et de se rapprocher du rouleau (12) au niveau de la position prédéterminée, les patins respectifs (9) du dispositif de rotation (1) présentant des vitesses circonférentielles variables ;
dans lequel les patins respectifs (9) du dispositif de rotation (1) reçoivent la deuxième bande (W2) au niveau d'une position de réception en amont de la position prédéterminée, un intervalle entre les patins adjacents (9) étant réduit après la réception, pendant la rotation des patins (9) vers la position prédéterminée, la partie desserrée étant ainsi formée sur la deuxième bande (W2) ;
dans lequel les premières bandes (W1) sont placées de sorte à être empilées sur une surface de la deuxième bande (W2), sur une base des patins (9), au niveau de la position prédéterminée ;
dans lequel un intervalle entre les patins adjacents (9) est accru pendant la rotation des patins respectifs (9) du dispositif de rotation (1) de la position prédéterminée vers une position de dégagement en aval de la position prédéterminée ; et
dans lequel le moyen de coupe (4) coupe en service l'élément élastique (F, F1, F2) dans la partie desserrée (Wa, Wb) pendant la rotation des patins (9) de la position prédéterminée vers la position de dégagement.
